# EUROPEAN PATENT APPLICATION

(11) **EP 4 610 276 A1**
(43) Date of publication of application: **03.09.2025**
(21) Application number: 23881442.0
(22) Date of filing: 28.08.2023
(51) Int. Cl.: C07K 14/765, C07K 1/18, C07K 1/14, A61K 38/00, A61K 38/38, A61P 7/00, C07K 14/76, C12N 15/14

(54) **METHOD FOR PREPARING HIGH-PURITY AND HIGH-STABILITY PROTEIN**

(30) Priority: 24.10.2022 CN 202211301410
(71) Applicant: Tonghua Anrate Biopharmaceutical Co., Ltd, Tonghua, Jilin 134000 (CN)
(72) Inventor: XIANG, Wei, Tonghua, Jilin 134100 (CN); YUE, Zhilei, Tonghua, Jilin 134100 (CN); DONG, Wenwen, Tonghua, Jilin 134100 (CN); GUAN, Tingchen, Tonghua, Jilin 134100 (CN)
(74) Representative: Winter, Brandl - Partnerschaft mbB
(86) International application number: PCT/CN2023/115220
(87) International publication number: WO 2024/087860

(57) **Abstract**

The present invention provides a process for obtaining high-purity and high-stability recombinant human albumin, wherein medium-long-chain fatty acid ligands are added and proteins of charge heterogeneity are removed through anion and/or cation chromatography. The process comprises the following steps. An inventiveness of the present invention is that the mixed solution contains a fatty acid mixture and poloxamer, and the molar ratio of oleic acid, myristic acid, sodium palmitate, sodium stearate to recombinant human albumin is 0.3:0.3:0.3:0.5:1. Sequential use of ion exchange chromatography can effectively remove truncated albumins, mismatched or modified albumins from recombinant albumins, to remove albumins of charge heterogeneity and modified albumins, to obtain high-purity and high-stability human albumin.

## Description

### FIELD OF THE INVENTION

The present invention relates to a process for obtaining high-purity recombinant human albumin by adding medium-long-chain fatty acid ligands and removing charge heterogeneity through anion or/and cation chromatography.

### DESCRIPTION OF RELATED ART

The human albumin have main pharmacological effects of regulating the dynamic balance of water between tissues and blood vessels, and maintaining normal and constant plasma volume. Furthermore, it also has high affinity for some ions and compounds, thus it can reversibly combine with these substances to play a transportation function. The human albumin can also provide a large amount of amino acids for the body.

Due to the above effects of the human albumin, it can be used in various clinical applications and therapies. Human albumin is mainly used to regulate plasma colloid osmotic pressure, expand blood volume, and treat wound, hemorrhagic shock, severe fire burn and hypoproteinemia. Furthermore, it is widely used in common diseases such as apoplexia, liver cirrhosis, liver ascites and kidney disease. In addition to being directly used in the clinical treatment, albumin has also been widely used in many applications such as the culture medium for vaccine production, pharmaceutical excipient, diagnostic reagent, new long-acting tumor pharmaceutical product, cosmetics and laboratory biological reagent.

The human albumin has a structure of a heart-shaped single chain non glycosylated protein with 585 amino acids, 17 pairs of disulfide bonds and a free sulfhydryl group. Its molecular weight is 66438 daltons. The half-life of human albumin in human body is 19-21 days. The heart-shaped structure of the human albumin consists of three main domains and six subdomains wrapped by 17 disulfide bonds, which are loosely bound together by van der Waals forces. It can be seen from its crystal structure that the disulfide bridge provides stiffness for the helical spherical structure, and also provides enough flexibility to enable the protein to make conformational changes according to the changes of the surrounding medium.

The conventional method for preparing human albumin comprises steps of extracting, isolating and purifying from human serum. The products are collectively named as human serum albumin. Because of the limited quantity of plasma sources, viral contamination of plasma donors and individual antibody and protein differences, the clinical applications of human albumin derived from human blood have risks. Therefore, there are viral safety statements in the manual of human albumin in many countries. For example, it is stated that "the standard treatments should be taken to prevent infection caused by the use of human blood or plasma products, including the selection of blood donors, the screening of a single blood supply or the screening of special infection markers in the plasma pool, and the effective production process to inactivate/remove the virus. Even so, when the medical products coming from blood or plasma are used, the possibility of infection by infectious pathogenic factors cannot be ruled out, because of unknown or new viruses and other pathogens". Therefore, gene recombination is the best way to effectively obtain virus-free albumin.

At present, yeast expression system is the most commonly used way to realize large-scale production of human albumin as the gene recombinant microorganisms express system, wherein Saccharomyces cerevisiae and Pichia pastoris are mostly used. However, because the human albumin belongs to a large volume injection preparation and the dose of each injection can be 5 grams to 30 grams, it must be required in each injection that the amount of the total host protein residue and pollutants in the production process shall not be greater than 1ng/ml (200mg/ml-rHA). No matter how the recombinant human albumin is produced, the glycosylation, oxidation, multimerization, mismatch, aggregation and other modifications of recombinant albumin may cause immunotoxicity to human body, and may also have a certain impact on the stability of the protein. Therefore, efficient and specific purification method is the key factor for obtaining high-purity homogenized recombinant human albumin.

The patent application WO1966/037515 (CN 1198844C) discloses a method of adding medium-short-chain fatty acids such as sodium octanoate before ion exchange chromatography, which can partly remove the albumins of charge heterogeneity after glycosylation modification. However, the ligand ability of sodium octanoate is weak, and it cannot effectively remove albumins of charge heterogeneous such as mismatch, oxidation and truncation.

The patent application CN2019108743433 discloses a method of adding medium-long-chain fatty acids or their salts and trace amounts of polymer excipients into recombinant albumin preparation to improve the stability of albumin preparation and prevent agglomeration and aggregation. However, this method cannot selectively remove hydrophobic heterogeneous albumin, charge-heterogeneous incomplete albumin, isomerised albumin or post modified albumin, and cannot remove a small amount of unstable heterogeneous albumin.

In the present invention, a certain amount of medium-long-chain fatty acids or their salts are added before pure ion exchange chromatography with reasonable ligand ratio to albumin, to improve the effective space folding of albumin to show the charge heterogeneity space of charge heterogeneous albumin. Under this condition, ion exchange chromatography can be used to effectively remove the albumins with fragments, mismatches and other incorrect stucture or post modification produced in fermentation and purification of recombinant albumin.

In the present description, the term "recombinant human albumin" can also be referred to as "recombinant albumin" and/or "recombinant human serum albumin" and/or "recombinant human blood albumin" and/or "rHA" and/or "rHSA". The term "human serum albumin" refers to human albumin extracted from human serum, which can also be called "human blood albumin" and/or "HSA" and/or "HA" and/or "pdHSA". In the present description, the term "medium-long-chain fatty acid" refers to a natural fatty acid or its salts with a carbon chain having more than 10 carbon atoms.

### SUMMARY OF THE INVENTION

A purpose of the present invention is to provide a better process for preparing high-purity and high-stability protein. The specific purpose is described in multiple substantive technical effects in the specific embodiments.

To achieve the above purpose, the following technical solutions are used in the present invention.

A process for preparing high-purity and high-stability protein, characterized in that, the process comprises the steps of:
step A comprising the substeps of:
   using a Millipore 10KDa membrane to replace the buffer solution of the recombinant human albumin collection, which is collected in previous purification, with an equilibrium solution of 80mM PB of pH 7.5, wherein a mixed solution is added into the collection solution, and the mixed solution contains a fatty acid mixture and poloxamer, and the molar ratio of oleic acid, myristic acid, sodium palmitate, sodium stearate to recombinant human albumin is 0.3:0.3:0.3:0.5:1; and
   loading and washing an anion exchange chromatography column, which has a column height of 380mm, with purified water to be neutral, and using an eluent of 50mM PB of pH 6.5 to elute the adsorbate in the chromatography column to collect recombinant human albumin component; and
step B comprising the substeps of:
   using 40mM HAc-NaOH equilibration liquid to equilibrate a cation exchange chromatography column, and using a Millipore 10KDa flat sheet membrane to perform desalting and liquid replacement to the collected recombinant human albumin component with a cation exchange chromatography equilibrium liquid, and loading and performing chromatography separation to collect recombinant human albumin component.

A process for preparing high-purity and high-stability protein, characterized in that, the process comprises the steps of:
step C comprising the substeps of:
   using a Millipore 10KDa membrane to replace the buffer solution of the recombinant human albumin collection, which is collected in previous purification, with an equilibrium solution of 40mM HAc-NaOH, and using a equilibrium solution to equilibrate a cation exchange chromatography column, and adding a mixed solution into the collection, and loading and performing chromatography separation to collect recombinant human albumin component, wherein the mixed solution contains a fatty acid mixture and poloxamer, and the molar ratio of oleic acid, myristic acid, sodium palmitate, sodium stearate to recombinant human albumin is 0.3:0.3:0.3:0.5:1;
step D comprising the substeps of:
   using a 10KDa Millipore membrane to replace the solution of recombinant human albumin component, which is collected in above step C, with a equilibrium solution as mentioned in step A, and using the equilibrium solution as mentioned in step A to equilibrate a anion exchange chromatography column, and using the same method as mentioned in step A to perform loading and purification to collect recombinant human albumin component.

In a preferred embodiment of the present invention, after step B and/or step D, the process further comprises the steps of: using a 100KDa and/or 30KDa and/or 10KDa membrane to intercept macromolecular aggregates and remove small molecular substances after the chromatography is finally completed, and performing replacement and concentrating to obtain recombinant human albumin stock solution with a concentration greater than 20%.

In a preferred embodiment of the present invention, the cation exchange medium includes but is not limited to the exchange medium of SP strong cation, or the CM weak cation. The cation exchange medium can be selected but is not limited to the Uni-SP/CM series, UniGel-SP/CM series, NanoGel SP series, Nano-SP series, Monomix-HC SP series, Monomix-MC SP series, GE's Sepharose series, or Bestarose series of Bestchrom Biotechnology Co., Ltd. The anion exchange medium includes but is not limited to the exchange medium of Q strong anion or the DEAE weak anion. The anion exchange medium can be selected from but is not limited to the Uni-DEAE/Q series, UniGel DEAE/Q series, NanoGel-Q series, Nano-Q series, Monomix-HC DEAE/Q series, Monomix-MC DEAE/Q series, GE's Sepharose series, or Bestarose series of Bestchrom Biotechnology Co., Ltd.

In a preferred embodiment of the present invention, the weight ratio of poloxamer to recombinant albumin is 10-500 micrograms of poloxamer per gram of recombinant albumin, and the poloxamer is used to promote the dissolution of fatty acids.

In a preferred embodiment of the present invention, purification of forward elution is used in the anion exchange chromatography, wherein the pH of the equilibrium solution and the eluent are configured to be between 6.0 and 9.5, and in the process of the present invention, the conductivity in the anion exchange chromatography is not higher than 20ms/cm; or, purification of reverse elution is used in the anion exchange chromatography, wherein the pH of the equilibrium solution is configured to be between 4.0 and 6.0, and the equilibrium conductance in anion exchange chromatography is not higher than 10 ms/cm.

In a preferred embodiment of the present invention, purification of forward elution is used in the anion exchange chromatography, wherein it is preferred that the pH of the equilibrium solution and the eluent are configured to be between 6.5 and 9.0, and the conductivity in the anion exchange chromatography is not higher than 15ms/cm; and purification of reverse elution is used in the anion exchange chromatography, and it is preferred that the pH of the equilibrium solution is configured to be between 4.0 and 5.5, and the equilibrium conductance in the anion exchange chromatography is not higher than 6 ms/cm.

In a preferred embodiment of the present invention, the pH of the equilibrium solution in the cation exchange chromatography is configured to be between 4.0 and 6.5, and the conductivity in the cation exchange chromatography is not higher than 10ms/cm.

In a preferred embodiment of the present invention, it is preferred that the pH of the equilibrium solution in the cation exchange chromatography is configured to be between 4.5 and 6.0, and the conductivity in the cation exchange chromatography is not higher than 5ms/cm.

In a preferred embodiment of the present invention, the recombinant human albumin collection, which collected in previous purification, is a human albumin product produced in large-scale production of gene recombinant microorganism express.

The present invention, which uses above technical solutions, has the following beneficial effects relative to the prior art. The first inventiveness of the present invention is that the molar ratio of oleic acid, myristic acid, sodium palmitate, sodium stearate to recombinant human albumin is 0.3:0.3:0.3:0.5:1. That is, the molar ratio of long-chain fatty acids or their salts to human recombinant albumin is 0.1~3:1. Sequential use of ion exchange chromatography can effectively remove truncated albumins, mismatched or modified albumins from recombinant albumins. These components may have side effects on human body or affect half-life and drug efficacy in clinic.

The second inventiveness of the present invention is that the medium-long-chain fatty acids or their salts are added before pure ion exchange chromatography, wherein the effective proportion of fatty acid ligands can be selected through ion exchange, and the components with too much or too little ligand proportion, which are generated in the course of hydrophobic heterogeneity due to albumin structural modification or misfolding, can be removed through ion exchange, so as to obtain high-purity and high-stability human albumin.

### BRIEF DESCRIPTION OF THE DRAWINGS

For the purpose of detailed description for the present invention, the following contents will be further described in combination with the drawings.
Fig.1 shows the comparison of charge heterogeneity detection maps between the sample before the treatment in Example 1 and the sample after sequential purification in Examples 1 and 2. In Fig.1, the ordinate is the absorption value (mAU), and the abscissa is the time axis (min).
Fig.2 shows the accelerated stability experiment, wherein DLS (dynamic light scattering instrument) is used to detect the particle size distribution of the samples at 0th, 1st and 3rd month. In Fig.2, the ordinate is the percentage of distribution, and the abscissa is the molecular diameter (nm);
Fig.3 shows the long term stability experiment, wherein DLS (dynamic light scattering instrument) is used to detect the particle size distribution of samples at 0th, 12th, 24th and 36th month. In Fig.3, ordinate is the percentage of distribution, and the abscissa is the molecular diameter (nm).

### DETAILED DESCRIPTION OF THE PRERERRED EMBODIMENTS

The present invention is further described below in combination with the drawings and embodiments. It should be understood that the following embodiments are only used to illustrate the present invention and not to limit the scope of the present invention.

The fermentation of the present invention is performed in an equipment of 10L, 20L, 3,000L or 10,0000L scale, and the purification is performed in a column with diameter of 10cm, 45cm and 120cm scale respectively, which has a good linear amplification performance. This example includes but is not limited to the above scales. The characteristics and advantages of the present invention can be further understood through the following examples in combination with the drawings, which does not limit the rest explanation contents of the present invention in any way.

### Example 1 Anion exchange chromatography

A Millipore 10KDa membrane is used to replace the buffer solution of the recombinant human albumin collection, which is collected in previous purification, with an equilibrium solution of 80mM PB of pH 7.5. A mixed solution, which contains oleic acid, myristic acid, sodium palmitate, sodium stearate and poloxamer, is added into the collection solution. The final concentration of fatty acids is 0.3:0.3:0.3:0.5:1 molar ratio (oleic acid: myristic acid: sodium palmitate: sodium stearate: recombinant human albumin). After loading, the UniGel-DEAE chromatography column (column height 380mm) is washed with purified water to be neutral. 50mM PB pH 6.5 eluent is used to elute the adsorbate in the chromatography column to collect the recombinant human albumin component.

### Example 2 Cation exchange chromatography

The SP Bestarose FF packing (equivalent to GE SP Sepharose Fast Flow packing) of Bestchrom (Shanghai) Biotechnology Co., Ltd. is used for cation exchange chromatography. 40mM HAc-NaOH equilibration liquid is used to equilibrate the chromatography column. A millipore 10KDa flat sheet membrane is used to perform desalting and liquid replacement to the collected recombinant human albumin components in Example 1 with the cation exchange chromatography equilibrium liquid. The loading and chromatography separation are performed to collect the recombinant human albumin component.

### Example 3 Cation exchange chromatography and anion exchange chromatography are exchanged

In this example, the process in Example 2 is performed first. A Millipore 10KDa membrane is used to replace the recombinant human albumin collection, which is collected in previous purification, with the buffer system of Example 2. A mixture solution of oleic acid, myristic acid, sodium palmitate, sodium stearate and poloxamer is supplemented. The final concentration of fatty acids is 0.3:0.3:0.3:0.5:1 molar ratio (oleic acid: myristic acid: sodium palmitate: sodium stearate: recombinant human albumin). The same method as in Example 2 is used to collect the recombinant human albumin component.

A Millipore 30KDa membrane is used to replace the above collected recombinant human albumin component with the equilibrium solution system of Example 1. The column is equilibrated. The same method as used in Example 1 is used to perform purification. Loading and collection of recombinant human albumin component are performed.

When the above chromatography is finally completed, 100KDa and/or 30KDa and/or 10KDa membrane can be used to intercept macromolecular aggregates and remove small molecular substances, and to perform replacement and concentrating to obtain recombinant human albumin stock solution with a concentration greater than 20%. Charge heterogeneity detection is performed on the sample before the treatment in Example 1 and the sample after sequential purification in Examples 1 and 2.

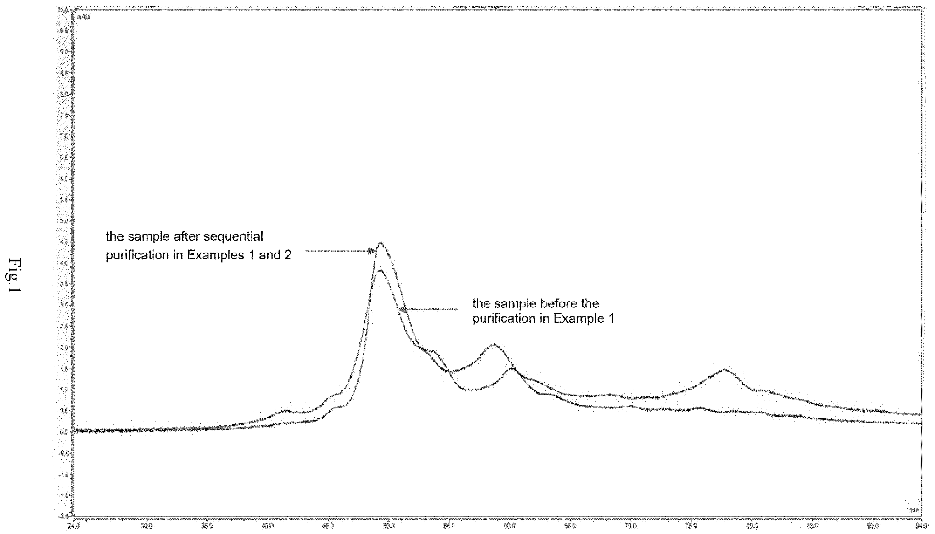

Regarding the detection method of charge heterogeneity, refer to 0513 Ion Chromatography Method of Volume IV General Principles of Chinese Pharmacopoeia 2020. The charge heterogeneity in recombinant human albumin is detected, and the detection results are as follows (Fig. 1).

### Example 4 Accelerated stability experiment

The protein solution purified in example 2 is used in this example. After the final production of the stock solution, the recombinant human albumin injection preparation is prepared. Under the conditions of temperature 25 °C ± 2 °C and humidity 60% ± 5%, the injection samples are tested by accelerated experiment. Generally, the test is performed for 6 months. The test results (Table 1, Table 2) and drawings (Fig. 1) are provided.

### Example 5 Long term stability experiment

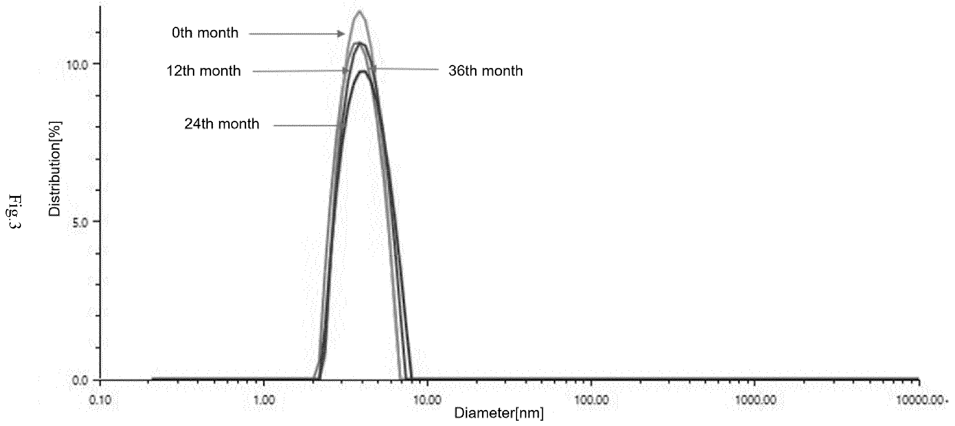

The protein solution purified in Example 2 is used in this example. After the final production of the stock solution, the recombinant human albumin injection preparation is prepared. Under the conditions of temperature 25°C ± 3°C, the injection samples are tested by long term stability experiment. The test is generally performed at time of: every three months in the first year, such as 0th, 3rd, 6th, 9th and 12th month, and every six months in the second year, such as 18th and 24th month, and every year, such as 36th month, after that. Test results (Table 3, Table 4), drawings (Fig. 3) are provided.

**Test results Table 1**

| Time | Appearance | Purity Native-PAGE | Polymer HPLC-SEC |
|---|---|---|---|
| 1st month | Slightly viscous, yellow brown clear liquid, no turbidity observed | 100% | 0.7% |
| 2nd month | Slightly viscous, yellow brown clear liquid, no turbidity observed | 100% | 0.7% |
| 3rd month | Slightly viscous, yellow brown clear liquid, no turbidity observed | 100% | 0.7% |
| 6th month | Slightly viscous, yellow brown clear liquid, no turbidity observed | 100% | 0.8% |

**Table 2: Changes of Tₒₙₛₑₜ, Tₘ₁, Tₘ₂ of samples at 0th, 3rd month in DSC detection.**

| Sample sequence | Tₒₙₛₑₜ (°C) | Tₘ₁ (°C) | Tₘ₂ (°C) |
|---|---|---|---|
| 0th month | 63.54 | 68.52 | 74.62 |
| 3rd month | 63.55 | 68.53 | 74.64 |

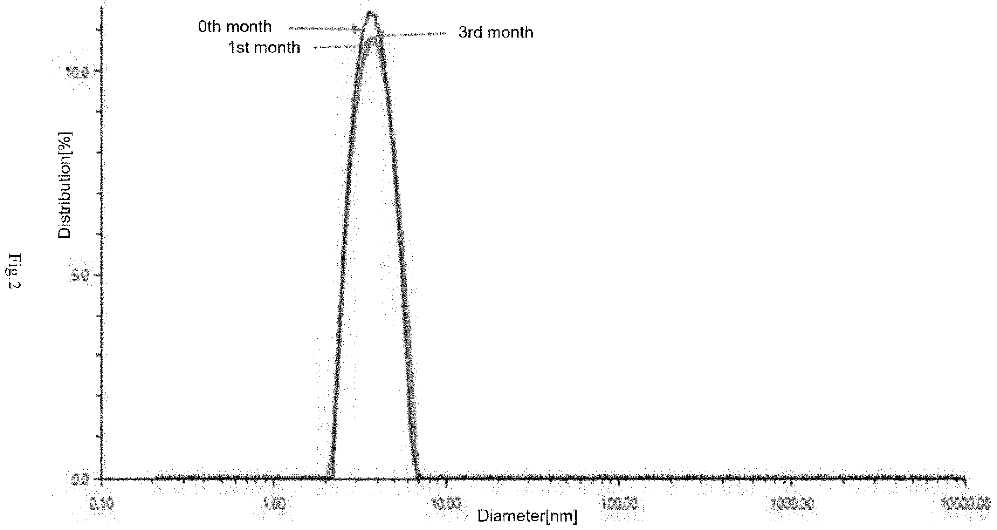

Fig. 2 shows the particle size distribution of the samples at 0th, 1st month, and 3rd month in DLS detection. After accelerated stability experiments, there was no significant change in the particle size distribution of the samples.

**Test Results Table 3:**

| Time | Appearance | Purity Native-PAGE | Polymer HPLC-SEC |
|---|---|---|---|
| 3rd month | Slightly viscous, yellow brown clear liquid, no turbidity observed | 100% | 0.7% |
| 6th month | Slightly viscous, yellow brown clear liquid, no turbidity observed | 100% | 0.7% |
| 9th month | Slightly viscous, yellow brown clear liquid, no turbidity observed | 100% | 0.7% |
| 12th month | Slightly viscous, yellow brown clear liquid, no turbidity observed | 100% | 0.7% |
| 18th month | Slightly viscous, yellow brown clear liquid, no turbidity observed | 100% | 0.7% |
| 24th month | Slightly viscous, yellow brown clear liquid, no turbidity observed | 100% | 0.7% |
| 36th month | Slightly viscous, yellow brown clear liquid, no turbidity observed | 100% | 0.8% |

**Table 4: Changes of Tₒₙₛₑₜ, Tₘ₁, Tₘ₂ of samples at 0th, 36th month in DSC detection.**

| Sample sequence | Tₒₙₛₑₜ (°C) | Tₘ₁ (°C) | Tₘ₂ (°C) |
|---|---|---|---|
| 0th month | 63.54 | 68.52 | 74.62 |
| 36th month | 63.56 | 68.55 | 74.66 |

Figure 3 shows the particle size distribution of the samples at 0th, 12th month, 24th month and 36th month in DLS detection. After long term stability experiment, there was no significant change in the particle size distribution of the samples.

In general, the present invention provides a process comprising steps of adding a certain amount of medium-long-chain fatty acids and their salts into purification intermediate product of recombinant albumin, and performing ion exchange chromatography to remove the proteins with fragments and incorrect-structure such as mismatch, which are generated in the course of fermentation and purification of the recombinant albumin. **In** the process of the present invention, the purified recombinant human albumin solution is further purified by means of cation exchange chromatography, anion exchange chromatography, and other methods.
1) Anion exchange chromatography;
2) Cation exchange chromatography.

The cation exchange medium adopted in the present invention is the SP series, or the CM series cation exchange medium, which can be selected but not limited to the Uni-SP/CM series, UniGel-SP/CM series, NanoGel SP series, Nano-SP series, Monomix-HC SP series, Monomix-MC SP series, GE's Sepharose series, or Bestarose series of Bestchrom Biotechnology Co., Ltd.

The medium for the anion exchange chromatography adopted in the present invention is the DEAE series or the Q series of strong anions. The anion exchange medium can be selected from but is not limited to the Uni-DEAE/Q series, UniGel DEAE/Q series, NanoGel-Q series, Nano-Q series, Monomix-HC DEAE/Q series, Monomix-MC DEAE/Q series, GE's Sepharose series, or Bestarose series of Bestchrom Biotechnology Co., Ltd.

Long-chain fatty acids can be added before anion exchange chromatography or be supplemented before cation exchange chromatography.

In the present invention, the buffer solution replacement and concentrating between the chromatography steps can be performed by hollow fiber membrane and flat sheet membrane with separation pore sizes ranging from 1kDa to 30KDa and other devices and equipments, including but being not limited to sequential and cross use.

In the present invention, the buffer solution replacement between the chromatography steps can also be treated with Sephadex G25 or Superdex G75 for replacement.

The recombinant albumin preparation with high charge consistency and hydrophobic consistency, which is obtained in the embodiments of the present invention, can be stored stably for 50 hours at 57°C according to the prescriptions of the Chinese Pharmacopoeia (2020 edition) or the United States Pharmacopoeia (USP39), and can also be stored stably for more than 30 days at 25°C or 30°C.

The poloxamer described in the present invention is selected from one or more of poloxamer 124, poloxamer 188, poloxamer 237, poloxamer 338, and poloxamer 407.

The high-purity recombinant human albumin, which is obtained through the process of the present invention, can be applied to same clinical indications as the ones to which the serum albumin derived from human blood is applied.

The high-purity recombinant human albumin, which is obtained through the process of the present invention, can be applied in diagnostic reagents, culture media, and pharmaceutical excipients.

The separation medium used in the present invention includes but is not limited to ion exchange mediums of hydrophilic-modified grafted microspheres between 10 µ m and 150 µ m, which are composed of polymers of polyacrylic acid ester or polystyrene-divinylbenzene. The separation medium can also be Sepharose 6 FF, or any one of above two polymer substrates and the agarose matrix, with length of the grafted side chains.

The height of column bed adopted in present invention is between 100mm and 800mm, preferably between 250mm and 600mm.

The chromatographic conditions in the present invention can be adjusted and modified according to a general instruction manual.

In the present invention, other conventional methods such as dialysis, ultrafiltration, and pasteurization can be used between the chromatographic chromatography, without affecting the effects of the present invention.

After the chromatography of the present invention is finally completed, a membrane of 100KDa, and/or 30KDa, and/or 10KDa can be used to intercept large molecule aggregates and remove small molecule substances, and to perform replacement and concentrating to obtain recombinant human albumin stock solution with a concentration greater than the preparation concentration.

The expression host cell described in the present invention is yeast, including Saccharomyces cerevisiae of Saccharomyces genus, Kluyveromyces genus, Hansenula genus, and Pichia genus.

The above content shows and describes the basic principles, main features, and advantages of the present invention. The skilled in the art should understand that the present invention is not limited by the above embodiments. The above embodiments and the description only illustrate the principles of the present invention. Without departing from the spirit and scope of the present invention, there may be various changes and improvements to the present invention, all of which fall within the scope of the claimed protection.

## Claims

1. A process for preparing high-purity and high-stability protein, **characterized in that**, the process comprises the steps of:
step A comprising the substeps of:
using a 10KDa membrane to replace the buffer solution of the recombinant human albumin collection, which is collected in previous purification, with an equilibrium solution of 80mM PB of pH 7.5, wherein a mixed solution is added into the collection solution, and the mixed solution contains a fatty acid mixture and poloxamer, and the molar ratio of oleic acid, myristic acid, sodium palmitate, sodium stearate to recombinant human albumin is 0.3:0.3:0.3:0.5:1, and the weight ratio of poloxamer to recombinant albumin is 10-500 micrograms of poloxamer per gram of recombinant albumin, and the poloxamer is used to promote the dissolution of the fatty acids; and
loading and washing an anion exchange chromatography column, which has a column height of 380mm, with purified water to be neutral, and using an eluent of 50mM PB of pH 6.5 to elute the adsorbate in the chromatography column to collect recombinant human albumin component; and
step B comprising the substeps of:
using 40mM HAc-NaOH equilibration liquid to equilibrate a cation exchange chromatography column, and using a 10KDa flat sheet membrane to perform desalting and liquid replacement to the collected recombinant human albumin component with a cation exchange chromatography equilibrium liquid, and loading and performing chromatography separation to collect recombinant human albumin component.

2. The process for preparing high-purity and high-stability protein according to claim 1, **characterized in that**, the process comprises the steps of:
step C comprising the substeps of:
using a 30KDa membrane to replace the buffer solution of the recombinant human albumin collection, which is collected in previous purification, with an equilibrium solution of 40mM HAc-NaOH, and using a equilibrium solution to equilibrate a cation exchange chromatography column, and adding a mixed solution into the collection, and loading and performing chromatography separation to collect recombinant human albumin component, wherein the mixed solution contains a fatty acid mixture and poloxamer, and the molar ratio of oleic acid, myristic acid, sodium palmitate, sodium stearate to recombinant human albumin is 0.3:0.3:0.3:0.5:1;
step D comprising the substeps of:
using a 10KDa membrane to replace the solution of recombinant human albumin component, which is collected in above step C, with a equilibrium solution as mentioned in step A, and using the equilibrium solution as mentioned in step A to equilibrate a anion exchange chromatography column, and using the same method as mentioned in step A to perform loading and purification to collect recombinant human albumin component.

3. The process for preparing high-purity and high-stability protein according to claim 1 or 2, **characterized in that**, after step B and/or step D, the process further comprises the steps of: using a 100KDa and/or 30KDa and/or 10KDa membrane to intercept macromolecular aggregates and remove small molecular substances after the chromatography is finally completed, and performing replacement and concentrating to obtain recombinant human albumin stock solution with a concentration greater than 20%.

4. The process for preparing high-purity and high-stability protein according to claim 1, **characterized in that**,
purification of forward elution is used in the anion exchange chromatography, wherein the pH of the equilibrium solution and the eluent are configured to be between 6.0 and 9.5, and in the process of the present invention, the conductivity in the anion exchange chromatography is not higher than 20ms/cm; or,
purification of reverse elution is used in the anion exchange chromatography, wherein the pH of the equilibrium solution is configured to be between 4.0 and 6.0, and the equilibrium conductance in the anion exchange chromatography is not higher than 10 ms/cm.

5. The process for preparing high-purity and high-stability protein according to claim 1, **characterized in that**, the pH of the equilibrium solution in the cation exchange chromatography is configured to be between 4.0 and 6.5, and the conductivity in the cation exchange chromatography is not higher than 10ms/cm.

6. The process for preparing high-purity and high-stability protein according to claim 1, is **characterized in that**, the recombinant human albumin collection, which collected in previous purification, is a human albumin product produced in large-scale production of gene recombinant microorganism express.
